(19) Europäisches Patentamt
European Patent Office
Office européen des brevets

(11) Publication number : 0 465 164 A1

# (12) EUROPEAN PATENT APPLICATION

(21) Application number : 91305889.7

(22) Date of filing : 28.06.91

(51) Int. Cl.[5] : C07H 19/09

(30) Priority : 30.06.90 GB 9014618

(43) Date of publication of application : 08.01.92 Bulletin 92/02

(84) Designated Contracting States : AT BE CH DE DK ES FR GB GR IT LI LU NL SE

(71) Applicant : THE WELLCOME FOUNDATION LIMITED
Unicorn House 160 Euston Road
London NW1 2BP (GB)

(72) Inventor : Gillies, Iain
Temple Hill
Dartford, Kent DA1 5AH (GB)
Inventor : Slucock, Keith Alan
Temple Hill
Dartford, Kent DA1 5AH (GB)

(74) Representative : Garrett, Michael et al
The Wellcome Research Laboratories Group
Patents and Agreements Langley Court
Beckenham Kent BR3 3BS (GB)

## (54) Process for the preparation of pyrimidine nucleosides.

(57) A process is provided for the preparation of a compound of formula (I) or a salt thereof :

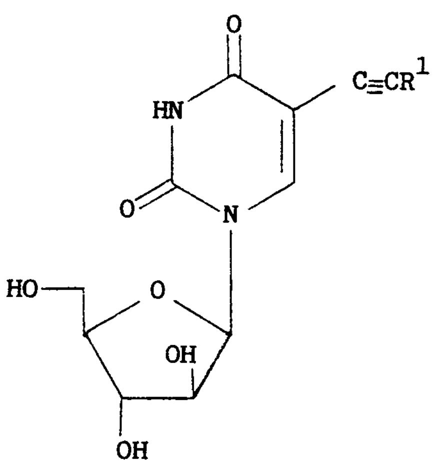

(I)

wherein $R^1$ is hydrogen or a $C_{1-4}$ alkyl, comprising reacting a compound of formula II :

EP 0 465 164 A1

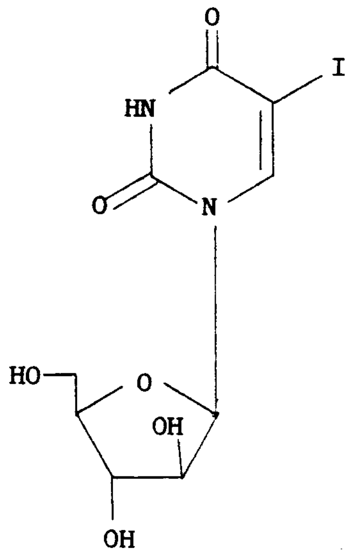

(II)

with an agent serving to introduce the alkynyl radical of formula $-C{\equiv}CR^1$ (wherein $R^1$ is as described above) at the 5-position of the pyrimidine base, the reaction being carried out in the presence of an $N_{1-6}$ alkylmorpholine, wherein said alkyl moiety being optionally substituted by an alkoxy or halogen group ; and optionally thereafter converting into a salt.

This invention concerns a process for the preparation of pyrimidine nucleosides, in particular 5-alkynyl substituted nucleosides such as, 1-(β-D-arabinofuranosyl)-5-prop-1-ynyluracil.

Tetrahedron Vol 43 No. 20 (1987) pp4601-8 discloses the preparation of (E)-5-(2-carbomethoxyvinyl)-2′-deoxyuridine from 2′-deoxy-5-iodouriine as an intermediate in the preparation of (E)-5-(2-bromovinyl)-2′-deoxyuridine. Nucleic Acids Research Vol 15(16) (1987) discloses the preparation of 5-(3-phthalimidopropynyl)-2′-deoxyuridine from 2′-deoxy-5-iodouridine.

1-(β-D-arabinofuranosyl)-5-prop-1-ynyluracil is disclosed in European Patent Publication No. 272068 for its use in the treatment of herpes viral infections particularly those infections caused by varicella zoster virus the causative agent of shingles and chicken pox. The European Patent Publication discloses various methods for the preparation of 1-(β-D-arabinofuranosyl)-5-(prop-1-ynyl)uracil including reaction of 1-(β-D-arabinofuranosyl)-5-iodouracil with an appropriate agent or agents to protect the hydroxy groups of the sugar moiety followed by the introduction of the desired propynyl group. De-protection of the hydroxy groups must then be carried out to provide the unprotected nucleoside.

A first feature of the invention provides a process for the preparation of certain 5-alkynyl pyrimidine arabino-nucleosides which involves a single stage reaction from a 5-iodo analogue as described below. This process requires no protection of the hydroxy groups of the sugar moiety. A second feature of the invention provides a process in which the yield of the final product from the starting material uridine, is greater than 23%. A third feature of the invention provides a process in which the purity of the final product is greater than 99%. A fourth feature of the invention provides a process which may be carried out at a temperature in the range 10-30°C.

This invention provides a process for the preparation of a compound of formula I or a salt thereof:

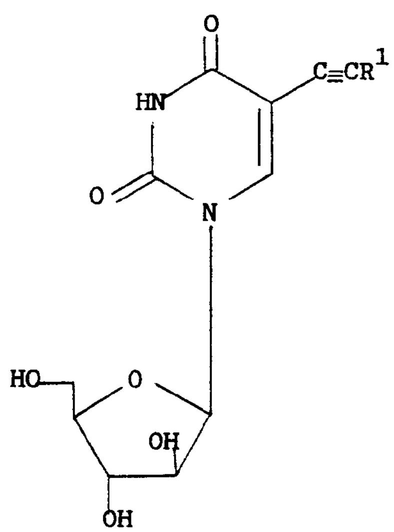

(I)

wherein $R^1$ is hydrogen or $C_{1-4}$ alkyl, comprising;
reacting a compound formula II:

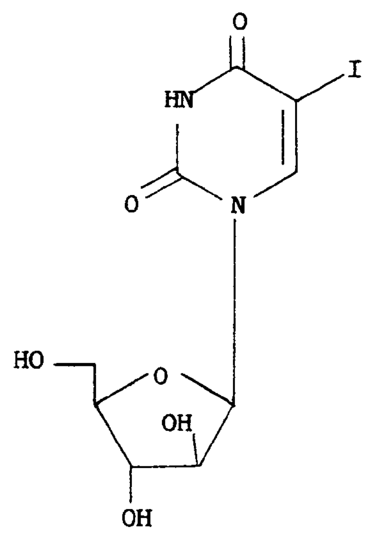

(II)

with an agent serving to introduce the alkynyl radical of formula -C≡CR[1] (wherein R[1] is as described above) at the 5-position of the pyrimidine base, wherein the reaction is carried out in the presence of an $N_{1-6}$ alkylmorpholine or said alkyl moiety being optionally substituted by an alkoxy (such as a $C_{1-6}$alkoxy more particularly $C_{1-4}$alkoxy) or halogen group; and optionally thereafter or simultaneously therewith, where the resulting compound is a compound of formula (I) converting it into a salt thereof, or where the resulting compound is a salt converting it into a different salt or a compound of formula (I).

The said alkyl moiety of the morpholine may be straight, branched or cyclic.

A compound of formula I may be prepared from a compound of formula II for example by reaction with said agent in the form of a gas or a liquid, in the presence of a catalyst, and optionally a co-catalyst, in solvent selected preferably from a $N_{1-6}$alkylmorpholine, more preferably from a $N_{1-4}$alkylmorpholine, conveniently at a temperature in the range 10-30°C.

The agent can be any appropriate alkynyl group (either protected or unprotected) which has a terminal alkynyl proton. The agent is for example, TMS acetylene (protected) for the introduction of an ethynyl group, propyne gas (unprotected) for the introduction of a propynyl group or butyne gas (unprotected) for the introduction of a butynyl group. The preferred catalysts are palladium (II) salts, such as palladium acetate or palladium chloride, optionally in the presence of, or in complex with, an aromatic ligand, such as triphenylphosphine, tetraphenylphosphine-0 or tri-(0-tolyl)phosphine, for example bistriphenylphosphine palladium (II) chloride. The preferred co-catalysts are metal anions for example copper (I) anions such as copper halides, specifically copper iodide, chloride or bromide. Most preferred catalysts and co-catalysts are palladium (II) acetate complexed with triphenylphosphine, and copper (I) iodide. The preferred solvents are N-methylmorpholine and more preferably N-ethylmorpholine and N-propylmorpholine. The preferred temperature for the reaction is in the range 20-25°C.

The preparation of a compound of formula I wherein R[1] is a propynyl group, may be effected as follows: the agent is propyne gas in the presence of palladium (II) acetate, triphenylphosphine and copper (I) iodide in N-ethylmorpholine at about 25°C.

A reference to a compound of formula I or II includes reference to a tautomeric form thereof.

The following examples are for illustration of the invention and should not be considered as limiting in any way:-

Example I

1-(β-D-Arabinofuranosyl)-5-(prop-1-ynyl)uracil

a) 2,2′-Anhydro-1-(β-D-arabinofuranosyl)uracil

Uridine (10g, 0.04mole) was dissolved in 20ml of warm, dry dimethylformamide, and 10cm³ of diethylcarbonate (0.08mole) and 0.2g of sodium bicarbonate were added. The solution was stirred and heated at 130°C until distillation of the ethanol by-product ceased (2.5 hours approximately). After cooling, the solution was diluted with 20cm³ of methanol with rapid stirring. The resulting solid was filtered off, and washed with methanol,

to give the title compound, as white crystals, melting at 235-240°C.

b) 1-(β-D-Arabinofuranosyl)uracil

The product of stage a) (7.0g, 0.03mole) was dissolved in 14cm$^3$ of 2.5M sodium hydroxide. After stirring at room temperature for 3.0 hours the solution was acidified to pH4-5 by portionwise addition of 80% acetic acid. After stirring for 1 hour at 5°C, the solid was filtered off and washed with 100ml of ethanol/water to give 1-(β-D-arabinofuranosyl)uracil, as white crystals, melting at 220-223°C.

c) 1-(β-D-Arabinofuranosyl)-5-iodouracil

The product of stage b) (3.0g, 12.3mmole), 1.6g of iodine (11.8mmole), and 12ml of 1.5M nitric acid were vigorously stirred together for 4.0 hours at 60°C. After cooling, a crystalline solid separated, which was filtered off, and washed thoroughly with ether to remove excess iodine. The solid was recrystallised from water to give 1-(β-D-arabinofuranosyl)-5-iodouracil as white crystals melting at 191-193°C (decomp).

d) 1-(β-D-Arabinofuranosyl)-5-(prop-1-ynyl)uracil

The product of stage c) (3.7g, 0.01mole) was mixed with N-ethylmorpholine (45ml), palladium (II) acetate 0.045g, 0.2mmol, triphenylphosphine 0.1g, 0.4mmol, and copper (I) iodide, 0.19g, 1mmol) with stirring. Propyne gas (0.8g 0.02mol) was added, and the mixture stirred for 48 hours at 20-25°C.

The reaction mixture was rotary evaporated to leave a yellow tacky solid which was partitioned between ethyl acetate (20cm$^3$) and water (30cm$^3$). The organic phase was discarded and the aqueous phase washed with ethyl acetate (2x20cm$^3$). The aqueous phase was neutralised with hydrochloric acid to give a white precipitate of 1-(β-D-arabinofuranosyl)-5-prop-1-ynyluracil, which was filtered off from the cooled filtrate.

| | |
|---|---|
| CHN Calculated: | C,51.06; H,4.964; N,9.93% |
| Found: | C,50.80; H,5.055; N,9.80% |

Mpt. = 225-227°C

Yield from uridine 24%

$^1$HNMR δ($d_6$DMSO) 11.52(1H,bs,NH), 7.8(1H,s,H-6), 5.95(1H,d,H-1′), 5.65-5.0(3H,m,OH-2′,OH-3′,OH-5′), 4.07-3.83(2H,m,H-2′,H-3′), 3.75(1H,m,H-4′), 3.59(2H,m,H-5′), 1.98 (3H,s,$CH_3$).

Example 2

Sodium salt of 1-(β-D-Arabinofuranosyl)-5-(prop-1-ynyl)uracil

A suspension of sodium hydride (0.05g of 80% w/v suspension in oil, 1.66mmol, washed several time with dry tetrahydrofuran) in dry tetrahydrofuran (4ml) was added to a stirred solution of 1-(β-D-arabinofuranosly)uracil (0.06g, 1.64mmol) in dry tetrahydrofuran, ensuring complete exclusion of moisture. The solvent was evaporated after 1 hour to give 0.1g of the required sodium salt. From this salt, the i.v. and opthalmic formulations were made up.

Examples 3 and 4

Step d) of Example 1 was repeated using N-methylmorpholine and N-propylmorpholine. The CHN, Mpt and HNMR were the same as above. For N-methylmorpholine the yield was 14% from uridine and for N-propylmorpholine the yield was 24% from uridine.

## Claims

**1.** A process for the preparation of a compound of formula (I) or a salt thereof:

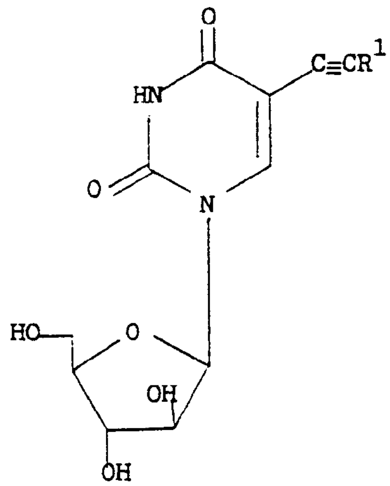

(I)

wherein $R^1$ is hydrogen or a $C_{1-4}$ alkyl, comprising reacting a coumpound of formula (II):

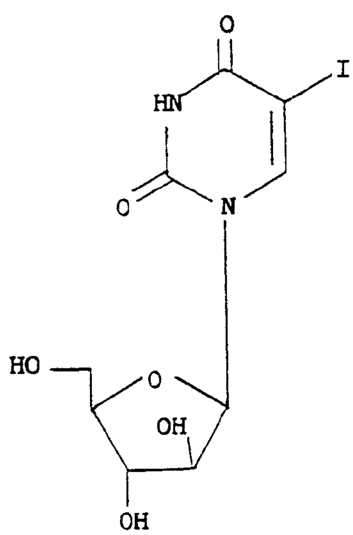

(II)

with an agent serving to introduce the alkynyl radical of formula $-C\equiv CR^1$ (wherein $R^1$ is as described above) at the 5-position of the pyrimidine base, wherein the reaction is carried out in the presence of an $N_{1-6}$ alkylmorpholine said alkyl moiety being optionally substituted by an alkoxy or halogen group; and optionally thereafter or simultaneously therewith, where the resulting compound is a compound of formula (I) converting it into a salt thereof, or where the resulting compound is a salt converting it into a different salt or a compound of formula (I).

2. A process as claimed in claim 1 wherein said morpholine is an unsubstituted $N_{1-6}$ alkylmorpholine.

3. A process as claimed in claim 2 wherein said morpholine is an unsubstituted $N_{1-4}$ alkylmorpholine.

4. A process as claimed in claim 3 wherein the alkyl moiety of the morpholine is ethyl.

5. A process as claimed in any preceding claim wherein $R^1$ is a methyl.

6. A process as claimed in any preceding claim wherein the agent serving to introduce the alkynyl radical is TMS acetylene, propyne gas, or butyne gas.

7. A process as claimed in any one of the preceding claims which is further carried out in the presence of a palladium (II) catalyst and a phosphine ligand.

8. A process as claimed in claim 7 wherein the palladium (II) catalyst is palladium acetate and the phosphine ligand is triphenylphosphine.

9. A process as claimed in claims 7 and 8 which is further carried out in the presence of a copper (I) anion.

10. A process as claimed in claim 9 wherein the copper (I) anion is selected from the group consisting of copper iodide, copper chloride, or copper bromide.

11. A process as claimed in any one of the preceding claims which is carried out at a temperature in the range 10 to 30°C.

European Patent Office

# EUROPEAN SEARCH REPORT

Application Number

EP 91 30 5889

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | THE JOURNAL OF ORGANIC CHEMISTRY, vol. 54, no. 14, 7th July 1989, pages 3420-3422, American Chemical Society; F.W. HOBBS, Jr.: "Palladium-catalyzed synthesis of alkynylamino nucleosides. A universal linker for nucleic acids" * The whole document * | 1,9,10 | C 07 H 19/09 |
| A,D | EP-A-0 272 065 (THE WELLCOME FOUNDATION LTD) * Abstract; page 9, line 25-49; page 11, line 1 - page 12, line 4 * | 1,6-10 | |
| A | EP-A-0 356 166 (THE WELLCOME FOUNDATION LTD) * Page 7, line 1 - page 9, line 59 * | 1,7-10 | |
| A | CHEMICAL ABSTRACTS, vol. 101, no. 3, 16th July 1984, page 638, abstract no. 23865d, Columbus, Ohio, US; A. MATSUDA et al.: "Introduction of carbon substituents at C-2 position of purine nucleosides", & NUCLEIC ACIDS SYMP. SER. 1983, 12(SYMP. NUCLEIC ACIDS CHEM., 11TH) 5-8 * Abstract * | 1,7 | TECHNICAL FIELDS SEARCHED (Int. Cl.5) |
| | | | C 07 H 19/00 |
| A | TETRAHEDRON LETTERS, vol. 31, no. 6, 1990, pages 807-810, Pergamon Press, Oxford, GB; V. NAIR et al.: "Copper mediated reactions in nucleoside synthesis" * The whole document * | 1,6-10 | |
| Y | EP-A-0 219 876 (YAMASA SHOYU K.K.) * Abstract; page 5, line 1 - page 6, line 11; page 8, lines 5-19 * | 1,6-11 | |

The present search report has been drawn up for all claims

| Place of search | Date of completion of the search | Examiner |
|---|---|---|
| THE HAGUE | 02-10-1991 | SCOTT J.R.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)